# EUROPÄISCHE PATENTSCHRIFT

(11) **EP 1 545 417 B1**
(45) Veröffentlichungstag und Bekanntmachung des Hinweises auf die Patenterteilung: **18.01.2006**
(21) Anmeldenummer: 03807771.5
(22) Anmeldetag: 02.08.2003
(51) Int. Cl.: A61F 13/15

(54) **INKONTINENZWINDEL FÜR ERWACHSENE**
INCONTINENCE DIAPERS FOR ADULTS
COUCHE POUR ADULTES INCONTINENTS

(30) Priorität: 30.09.2002 DE 10246365
(43) Veröffentlichungstag der Anmeldung: 29.06.2005
(73) Patentinhaber: Paul Hartmann Aktiengesellschaft, 89522 Heidenheim (DE)
(72) Erfinder: RÖHRL, Wolfgang, 89542 Herbrechtingen (DE)
(74) Vertreter: Friz, Oliver
(86) Internationale Anmeldenummer: PCT/EP2003/008586
(87) Internationale Veröffentlichungsnummer: WO 2004/032812

(56) Entgegenhaltungen:
- EP-A- 0 000 969
- WO-A-00/35397
- US-A- 4 500 316
- US-A- 4 704 115
- US-B1- 6 307 120

## Beschreibung

Die Erfindung betrifft eine wegwerfbare Inkontinenzwindel für Erwachsene, mit einem Vorderbereich, einem Rückbereich und einem in einer Längsrichtung dazwischen liegenden im Schrittbereich des Benutzers zu liegen kommenden Mittelbereich, und mit mechanisch oder klebend wirkenden Verschlussmitteln, die an Seitenabschnitten, insbesondere an Längsrandabschnitten des Rückbereichs oder des Vorderbereichs angeordnet sind und zum Schließen der Windel mit einem Auftreffabschnitt am Vorderbereich oder Rückbereich haftend zusammenwirken, wobei an einem jeweiligen Seitenabschnitt oder Längsrandabschnitt wenigstens zwei Verschlussmittel vorgesehen sind, die in Längsrichtung voneinander beabstandet sind.

Eine derartige Windel ist in EP 0 000 969 Bl vorbeschrieben. Auch US 4,209,016 offenbart eine Windel mit Verschlussmitteln, die entweder geteilt oder teilbar ausgebildet sind, so dass neben der Primärschließfunktion durch einen dem Hüftrand nahen Tapestreifen ein zweiter Tapestreifen zur Erreichung zusätzlicher Spannkräfte, welche einen guten Sitz der Windel am Körper des Benutzers vermitteln sollen, unterhalb des ersten Tapestreifens positioniert werden kann.

Ferner offenbart WO 00/37005 eine gattungsgemäße Inkontinenzwindel mit beidseits wenigstens drei Verschlussmitteln.

Der vorliegenden Erfindung liegt die Aufgabe zugrunde, eine wegwerfbare Inkontinenzwindel der genannten Art dahingehend zu verbessern, dass sie besser an in einer Tragesituation auftretende Belastungen im Verschlussbereich angepasst ist.

Diese Aufgabe wird bei einer in Rede stehenden Inkontinenzwindel erfindungsgemäß dadurch gelöst, dass das jeweilige vom Hüftrand weiter entfernt angeordnete Verschlussmittel und der diesem zugeordnete Auftreffabschnitt so ausgebildet sind, dass sich ihre Haftverbindung ausgehend vom geschlossenen Zustand der Windel erst bei einer höheren Kraft löst als die Haftverbindung bei dem näher am Hüftrand vorgesehenen Verschlussmittel und seinem zugeordneten Auftreffabschnitt.

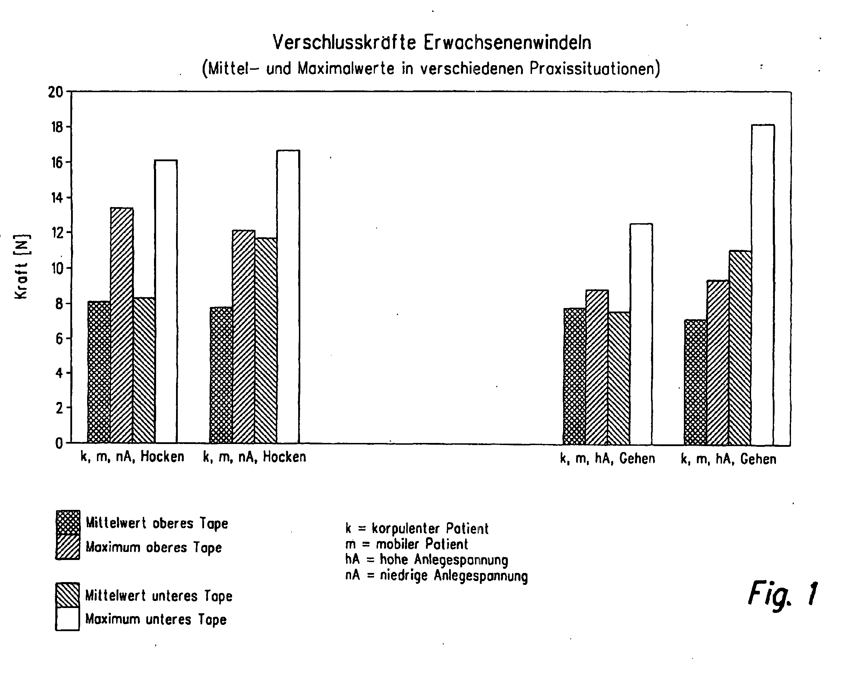

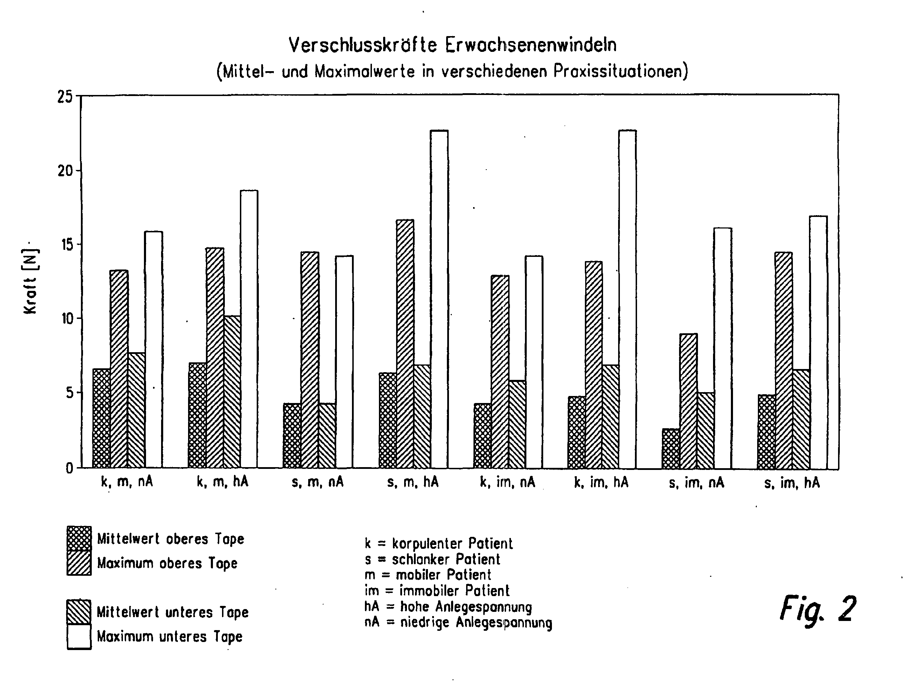

Die vorliegende Erfindung beruht auf der Erkenntnis, dass gerade bei Inkontinenzwindeln für Erwachsene, und zwar sowohl für mobile als auch immobile, pflegebedürftige Erwachsene, in der tatsächlichen Tragesituation größere Belastungen auf das hüftferne Verschlusssystem einwirken als auf das hüftnahe Verschlusssystem. Diese Belastungen treten auf in Form von Zugkräften in der flächenhaften Erstreckung des Verschlusssystems. Diesen überraschenden Sachverhalt hat die Anmelderin durch umfangreiche Messungen unter Verwendung von Dehnungsmessstreifen bei hüftnah und hüftfern, also in einer Tragesituation oberen und unteren Verschlusssystemen bei Inkontinenzwindeln im Versuch ermittelt. Die Ergebnisse solcher Messungen sind in den Figuren 1 und 2 dargestellt und werden an späterer Stelle erläutert werden.

Wenn seither bei der Konfiguration von Inkontinenzwindeln für Erwachsene hohe Belastungssituationen berücksichtigt werden sollten, so führte dies nicht selten zu einer rohstoffintensiven Überdimensionierung von Verschlussmitteln, die in Tapeform oder Laschenform mit klebenden oder mechanisch wirkenden Verschlusselementen verwirklicht wurden.

Mit der vorliegenden Erfindung wurde in Abkehr hiervon festgestellt, dass es ausreichend ist, das Verschlusssystem bei einer gattungsgemäßen Inkontinenzwindel auf die maximalen Belastungen im Bereich der hüftfern angeordneten Verschlussmittel abzustimmen und bei hüftnah angeordneten Verschlussmitteln eine demgegenüber schwächere Auslegung der Haftverbindung zwischen dem hüftnahen Verschlussmittel und dem korrespondierenden Auftreffabschnitt vorzusehen. Dies ermöglicht eine ökonomische und auch ökologische Optimierung des Rohmaterialeinsatzes.

Es sei darauf hingewiesen, dass es im allgemeinen nicht der besonderen Optimierung der in der Regel tapeförmigen oder laschenförmigen Verschlussmittel selbst bedarf, um Belastungskräfte zerstörungsfrei aufzunehmen; sie sind in der Regel hinreichend stabil und geeignet, die in einer Tragesituation im Gebrauch auftretenden Zugkräfte oder Zugbelastungen aufzunehmen. Vielmehr steht, wie vorstehend ausgeführt und beansprucht, die Ausbildung der Haftverbindung zwischen den Verschlussmitteln und den korrespondierenden Auftreffabschnitten im Vordergrund des Interesses. Es besteht nämlich die Gefahr, dass diese Haftverbindung sich bei besonderen Belastungssituationen löst. Durch die erfindungsgemäße Erkenntnis und den erfindungsgemäßen Vorschlag der Konfigurierung des Verschlusssystems wurde eine weitere Verbesserung von Inkontinenzwindeln im Hinblick auf die tatsächlich auftretenden Belastungen in einer Tragesituation, aber auch im Hinblick auf die Möglichkeit eines optimierten Rohmaterialeinsatzes erreicht.

Eine im Bereich der hüftfern angeordneten Verschlussmittel stärkere Haftverbindung lässt sich beispielsweise dadurch erreichen, dass das betreffende hüftfern angeordnete Verschlussmittel einen flächenmäßig größeren aktiven Haftbereich aufweist als das näher am Hüftrand angeordnete. Wenn solchenfalls insbesondere die Ausbildung der Verschlussmittel oder des aktiven Haftbereichs der Verschlussmittel und der zugeordneten Auftreffbereiche gleich sind, es sich also um dasselbe Haken/Schlaufen-Material (hook and loop) oder um dasselbe klebende Verschlusstapematerial handelt, so ergibt sich durch die flächenmäßig größere Ausbildung des aktiven Haftbereichs bei dem hüftfern angeordneten Verschlussmittel eine-stärkere Haftkraft als bei dem hüftnah angeordneten Verschlussmittel.

Die größere Fläche des aktiven Haftbereichs des weiter vom Hüftrand entfernt angeordneten Verschlussmittels beträgt dabei wenigstens das 1,2-Fache, insbesondere das 1,2 - 2-Fache und vorzugsweise bis höchstens das 1,6-Fache oder das 1,5-Fache der Fläche des aktiven Haftbereichs des näher am Hüftrand angeordneten Verschlussmittels.

Nach einer bevorzugten Ausführungsform der Erfindung ist die Abmessung des aktiven Haftbereichs des vom Hüftrand weiter entfernt angeordneten Verschlussmittels in Längsrichtung der Windel größer als die Abmessung des aktiven Haftbereichs des näher am Hüftrand angeordneten Verschlussmittels in dieser Längsrichtung. Es könnten solchenfalls also zur Ausbildung der hüftfernen und hüftnahen Verschlussmittel Verschlusstapes oder -laschen oder allgemein einen klebend oder mechanisch wirkenden aktiven Haftbereich aufweisende Verschlussmittel unterschiedlicher Abmessung in Längsrichtung der Windel verwendet werden, wobei insbesondere deren Abmessung in Querrichtung der Windel gleich sein kann.

Wenn vorstehend von einem Auftreffabschnitt für die jeweiligen Verschlusselemente die Rede ist, so ist dieser Auftreffabschnitt im weitesten Sinne zu verstehen. Es kann sich hierbei beispielsweise um die Backsheet-Folie der Inkontinenzwindel handeln, die einen Auftreffbereich für die Verschlussmittel bildet, der nicht einmal notwendigerweise in irgendeiner Weise gekennzeichnet sein muss. Es wäre auch denkbar, dass der jeweilige Auftreffabschnitt für die jeweiligen Verschlussmittel von einem einzigen oder mehreren Abschnitt(en) einer Flachmaterialware, beispielsweise eines Haken-Schlaufen-Materials oder eines eher glatten Materials zur Bildung einer klebenden Haftverbindung, gebildet ist. Insbesondere könnten die Auftreffabschnitte für das obere und untere Verschlussmittel an einem jeweiligen Seitenabschnitt der Windel von einem einzigen durchgehenden Auftreffabschnitt gebildet sein. In entsprechender Weise könnte sich ein Auftreffabschnitt von einem Seitenabschnitt zum anderen in Querrichtung erstrecken, was dann eine in Querrichtung weitgehend variable Positionierung der jeweiligen Verschlussmittel gestattet, um die Inkontinenzwindel an den jeweiligen Hüftumfang eines Benutzers und die gewünschte Anlegespannung anpassen zu können.

Nach einer weiteren Ausführungsform entspricht dabei die Abmessung des aktiven Haftbereichs des jeweiligen Verschlussmittels in Längsrichtung der Windel im wesentlichen der Abmessung dieses Verschlussmittels in dieser Richtung. Beispielsweise könnte also ein klebend ausgebildetes Verschlusstape über die gesamte Tapebreite (die dann bei beispielhafter Ausrichtung des Tapes in Querrichtung der Windel in deren Längsrichtung verläuft) mit einer über die gesamte Breite des Verschlusstapes erstreckten Kleberbeschichtung versehen sein.

Vorzugsweise ist die Abmessung des gesamten vom Hüftrand weiter entfernt angeordneten Verschlussmittels in Längsrichtung der Windel größer als die Abmessung des näher am Hüftrand angeordneten Verschlussmittels in dieser Richtung.

Wie schon erwähnt, kann die Abmessung des aktiven Haftbereichs des vom Hüftrand weiter entfernt angeordneten Verschlussmittels in Querrichtung der Windel im wesentlichen der Abmessung des aktiven Haftbereichs des näher am Hüftrand angeordneten Verschlussmittels in dieser Querrichtung entsprechen. Im Hinblick auf ein optisch ansprechendes Erscheinungsbild des Hygieneartikels werden auch die Abmessungen der jeweiligen Verschlussmittel als Ganzes in der Querrichtung der Windel vorzugsweise gleich gewählt.

Bevorzugte Abmessungen der Verschlussmittel ergeben sich aus den abhängigen Patentansprüchen.

Nach einer bevorzugten Ausführungsform der Erfindung sind die Verschlussmittel und die zugeordneten Auftreffbereiche so ausgebildet, dass die Ablösekraft, bei der sich die Haftverbindung zwischen dem weiter vom Hüftrand entfernt angeordneten Verschlussmittel und dem zugeordneten Auftreffabschnitt löst, wenigstens 45 N beträgt, wobei diese Ablösekraft als maximale Scherkraft bei einem Abzugsversuch zwischen dem Verschlussmittel und dem Auftreffabschnitt gemessen wird. Der Begriff der Scherkraft bedeutet dabei, dass einerseits das Verschlussmittel und andererseits der Auftreffabschnitt in einer noch zu beschreibenden Versuchsanordnung in einem Zugprüfgerät eingespannt und dann in einer Ebene und unter einem Abzugswinkel von im wesentlichen 0° auseinanderbewegt werden (bei der Bestimmung der Abschälkraft oder auch peel-Kraft würde das Verschlussmittel unter einem Abzugswinkel von im wesentlichen 180° abgezogen werden, so dass das Verschlussmittel nach und nach abgelöst wird). Bei der hier angesprochenen Bestimmung der maximalen Scherkraft wird das Verschlussmittel quasi schlagartig bei Erreichen der hierfür erforderlichen Ablösekraft vom Auftreffabschnitt, der durch ein Versuchssubstrat simuliert wird, abgelöst.

Die vorstehend erwähnte Ablösekraft beträgt bei dem hüftfern angeordneten Verschlussmittel und dem zugeordneten Auftreffabschnitt insbesondere höchstens 105 N, vorzugsweise zwischen 55 N und 95 N.

Die entsprechende Ablösekraft bei dem hüftnah angeordneten Verschlussmittel - wiederum gemessen als maximale Scherkraft - beträgt vorzugsweise wenigstens 38 N. Eine bevorzugte obere Grenze stellen 60 N dar. Die Ablösekraft liegt nach einer bevorzugten Ausführungsform zwischen 40 N und 55 N.

Weitere Merkmale, Einzelheiten und Vorteile der Erfindung ergeben sich aus den beigefügten Patentansprüchen und der zeichnerischen Darstellung und nachfolgenden Beschreibung von der Erfindung vorausgehenden Messungen der Belastungskräfte bei Inkontinenzwindeln, der erfindungsgemäßen Inkontinenzwindel und des Versuchsaufbaus zur Bestimmung der erwähnten Ablösekraft. Es sei darauf hingewiesen, dass für die jeweiligen Merkmale der Patentansprüche auch jeweils unabhängig von ihrer Rückbeziehung auf andere Patentansprüche selbständig Schutz in Anspruch genommen wird. In der Zeichnung zeigt:
- Figuren 1 und 2: bei verschiedenen Tragsituationen ermittelte Kräfte, die im Verschlusssystem von Inkontinenzwindeln auftreten;
- Figur 3: eine Draufsicht auf eine erfindungsgemäße Inkontinenzwindel in schematischer Darstellung und
- Figur 4: den Versuchsaufbau zur Bestimmung der maximalen Ablösekraft.

Die Figuren 1 und 2 zeigen das Ergebnis von Untersuchungen von Zugkräften, die bei verschiedenen Tragesituationen von Inkontinenzwindeln für Erwachsene im Bereich der Verschlussmittel gemessen wurden. Die Untersuchungen wurden anhand zweier Versuchspersonen durchgeführt. Dabei wurden eher große und schwere Versuchspersonen ausgewählt, die aber zusätzlich unterschiedlichen Konstitutionstypen, nämlich eher korpulent bzw. eher schlank, zugeordnet wurden. Es wurden des weiteren verschiedene Tragesituationen nachempfunden. Einmal (Figur 1) wurden mobile Patienten mit normalem Bewegungsablauf simuliert, und zwar während des Gehens und Hockens, als beispielhafter Bewegungszustände. Zudem wurde noch zwischen einer hohen Windelanlegespannung und einer niedrigen Windelanlegespannung unterschieden, die durch strammes oder straffes Schließen der Windel durch entsprechendes Positionieren der Verschlusselemente variiert werden kann (Figur 1 links und rechts). Es sind dann in einem jeweiligen Block in Figur 1 der Mittelwert und der Maximalwert der gemessenen Kraftwerte (Zugspannungskräfte) jeweils für das obere und für das untere tape-förmige Verschlussmittel angegeben.

Man erkennt, dass bei beiden Belastungssituationen, Hocken und Gehen, sowohl bei hoher als auch bei niedriger Anlegespannung die im oberen, also hüftnah angeordneten Verschlussmittel gemessenen Kräfte geringer sind als die im unteren, also hüftfern angeordneten Verschlussmittel gemessenen Kräfte.

Messtechnisch wurden die Zugkräfte dadurch ermittelt, dass in die jeweiligen tapeförmigen Verschlussmittel Dehnmessstreifen als spezielle Zugkraftsensoren integriert wurden.

Figur 2 zeigt das Ergebnis weiterer Zugspannungsmessungen im Verschlusssystem einer gattungsgemäßen Inkontinenzwindel für Erwachsene, wobei wiederum jeweils der Mittelwert und der Maximalwert von einer Mehrzahl von Messungen in verschiedensten Praxissituationen angegeben ist. Bei der Simulation mobiler Patienten sind diese verschiedenen Praxissituationen oder Tragesituationen gegeben u. a. durch Gehen auf einer Ebene, Treppensteigen, Bücken, Hocken, Vorbeugen, Hinlegen, Hin- und Herrutschen, Aufstehen. Bei der Simulation immobiler, pflegebedürftiger Patienten, die von einer Pflegeperson zumeist liegend manipuliert werden, sind diese Praxissituationen gegeben u. a. durch Anwinkeln einer oder zweier Beine, Hochstellen des Fußteils oder des Oberkörperteils eines klinischen Bettes, Drehen der Versuchsposition in 30°, 60°, 90° oder 135° Seitenlage und Zurückdrehen, Durchführen eines Bettwäschewechsels bei liegenden Patienten. Die jeweils dargestellten Blöcke zeigen wieder am Mittelwert und Maximalwert beim oberen (hüftnahen) und unteren (hüftfernen) Verschlussmittel, wobei zusätzlich zwischen den eingangs erwähnten Konstitutionstypen korpulent bzw. schlank unterschieden wird. Auch hier zeigen die Untersuchungen eindeutig, dass das untere, also hüftfern angeordnete Verschlusssystem, stärker beansprucht wird als das obere, also hüftnahe Verschlusssystem.

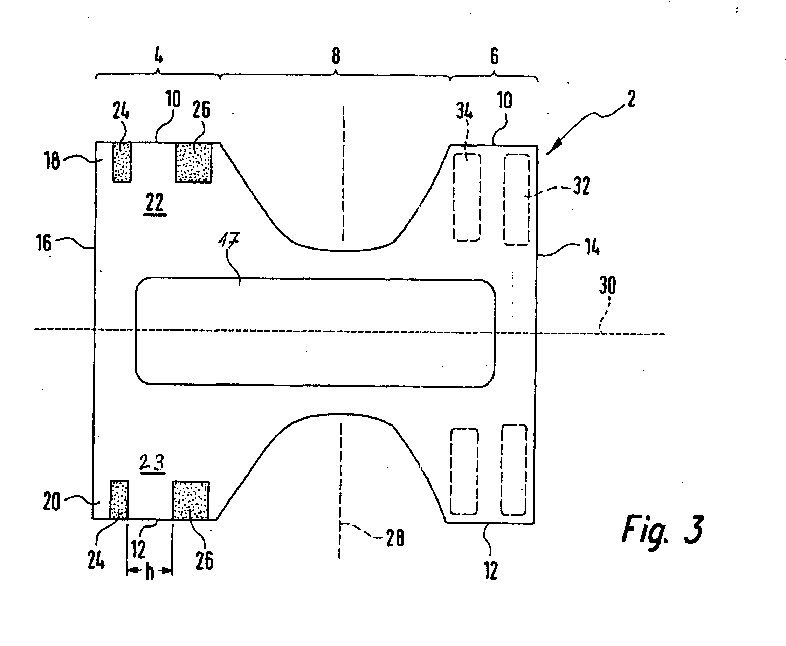

Figur 3 zeigt schematisch in der Draufsicht eine erfindungsgemäße Inkontinenzwindel 2 mit einem Rückbereich 4, einem Vorderbereich 6 und einem dazwischen liegenden Mittel- oder Schrittbereich 8 sowie mit Längsseitenrändern 10, 12 von Vorderbereich 6 und Rückbereich 4 und mit Hüfträndern 14, 16. Ferner ist ein verhältnismäßig schmaler Saugkörper 18 dargestellt. In Längsrandabschnitten 18, 20 eines jeweiligen Seitenabschnitts 22, 24 des Rückbereichs 4 sind ein hüftnah angeordnetes Verschlussmittel 24 und ein hüftfern angeordnetes Verschlussmittel 26 im Abstand h von 105 mm voneinander vorgesehen, die vorliegend auf die Sichtseite der Inkontinenzwindel 2 gefaltet sind. Sie lassen sich nach außen auffalten, so dass sie über den jeweiligen Längsrand 10, 12 in einer Querrichtung 28 der Windel 2 vorstehen. Sie könnten aber auch ganz innerhalb der Seitenabschnitte 22, 24 vorgesehen sein. Senkrecht zu dieser Querrichtung 28 verläuft eine Längsrichtung 30 der Inkontinenzwindel 2.

Die schematisch dargestellten Verschlussmittel 24, 26 können klebend oder mechanisch haftend, etwa mittels eines Haken/Schlaufenmaterials, ausgebildet sein. Es handelt sich hierbei insbesondere um Längsabschnitte eines in Längs- oder Querrichtung zugeführten endlosen Streifen- oder Bandmaterials, welche im "cut and place"-Verfahren im Rückenbereich 4 der Inkontinenzwindel 2 angeordnet werden.

Im dargestellten Fall mag die angedeutete flächenhafte Erstreckung der Verschlussmittel 24, 26 auch deren aktiven Haftbereich darstellen, also etwa eine vollflächige Kleberbeschichtung der Verschlussmittel, gegebenenfalls mit einem nicht beschichteten Anfassbereich, so dass man erkennt, dass die Fläche dieses aktiven Haftbereichs bei dem hüftfern angeordneten Verschlussmittel 26 größer ist als bei dem hüftnahen Verschlussmittel 24. Dies führt dann, insbesondere bei funktional gleich ausgebildeten Verschlusssystemen, erfindungsgemäß zu einer stärkeren Haftverbindung bei dem hüftfernen Verschlussmittel 26; die sich erst bei einer höheren Ablösekraft löst, als bei dem hüftnahen Verschlussmittel 24. Diese Haftverbindung ergibt sich, wenn die Windel 2 an einen Benutzer angelegt wird und die Verschlussmittel 24, 26 auf ihnen zugeordnete Auftreffbereiche 32, 34 im Vorderbereich 6 der Windel aufgebracht oder aufgedrückt werden. Es erweist sich als vorteilhaft und einfach, wenn die jeweiligen Auftreffbereiche 32, 34 gleich ausgebildet werden; sie können, insbesondere von der Oberseite eines Schichtmaterials der Windel gebildet werden, beispielsweise von einer Folienschicht der Windel, etwa aus Polyethylen oder aus Polypropylen. Es kann sich dabei aber auch um ein auf eine Windelseite aufgebrachtes Haken- oder Schlaufenmaterial handeln. Auf die eingangs gemachten Ausführungen zur Ausbildung der Auftreffabschnitte wird ausdrücklich hingewiesen. Wenn also die Verschlussmittel 24, 26 und die zugehörigen Auftreffbereiche 32, 34 funktional gleich ausgebildet sind, so führt - wie im dargestellten Fall - die größere flächenhafte Erstreckung des hüftfern angeordneten Verschlussmittels 26 zu einer stärkeren Haftverbindung. Anders ausgedrückt, kann mit der Erfindung erreicht werden, dass dort, wo hohe Belastungen auftreten, nämlich bei den hüftfern angeordneten Verschlussmitteln 26, eine entsprechende Dimensionierung der Verschlussmittel und/oder ihrer aktiven Haftbereiche vorgenommen wird, während bei hüftnah angeordneten Verschlussmitteln eine weniger aufwendige Dimensionierung der Verschlussmittel bzw. deren aktiver Haftbereiche hinreichend ist. Hierdurch kann der Rohmaterialeinsatz den Anforderungen entsprechend optimiert werden. Es sei aber darauf hingewiesen, dass die erfindungsgemäß vorgeschlagene unterschiedliche starke Ausbildung der Haftverbindung auch in anderer Weise als durch verschieden große Verschlussmittel oder verschieden große aktive Haftbereiche bei Verschlussmitteln erreicht oder unterstützt werden kann. Beispielsweise wäre es auch denkbar, das bei den hüftfern angeordneten Verschlussmitteln ein anderer stärker haftender Kleberauftrag oder eine zu einer stärkeren Haftverbindung führende Ausbildung des Verschlussmittels und/oder des zugeordneten Auftreffbereichs vorgesehen werden könnte.

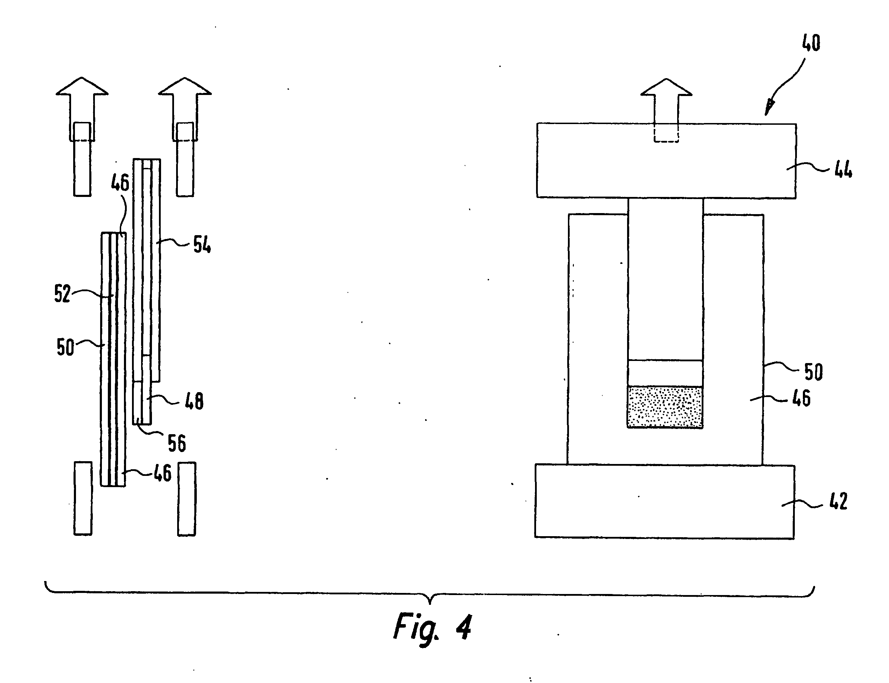

Wenn vorstehend von einer stärkeren Haftverbindung zwischen den hüftfern angeordneten Verschlussmitteln 26 und den diesen zugeordneten Auftreffbereichen 34 die Rede ist, so kann dies vorzugsweise durch Angabe der Ablösekräfte, und zwar gemessen als in einem Zugspannungstest auftretende Scherkräfte angegeben bzw. überprüft werden. Dies wird nachfolgend anhand des in Figur 4 schematisch dargestellten Versuchsaufbaus erläutert: Es wird mit dieser Prüfmethode die Verbundhaftkraft von Tapematerialien, welche ein Verschlussmittel simulieren, zu einem Testsubstrat, welches einen Auftreffbereich simuliert, bestimmt. Diese Verbundhaftkraft wird mittels einer Scherbeanspruchung in einem Zugprüfgerät ermittelt. Es handelt sich hierbei beispielsweise um ein schematisch angedeutetes Zugprüfgerät 40 nach EN ISO 527-1 (vom April 1996) mit einer feststehenden Klemme 42 und einer bewegbaren Klemme 44, die zum Einspannen des Testsubstrats 46 einerseits und des Verschlussmittel-Tapes 48 andererseits dient.

Zur Probenpräparation wird auf eine 55 mm breite Stahlplatte 50 mittels eines doppelseitigen Klebebands 52 das ebenfalls 55 mm breite Testsubstrat 46 aufgebracht. Es handelt sich bei dem Testsubstrat um einen Abschnitt einer üblichen Windel-Backsheet-Folie, beispielsweise in Form einer Polyethylenfolie von 21 *µ*m Dicke, der quer zur Laufrichtung ausgestanzt ist.

Der das Verschlussmittel darstellende streifenförmige Abschnitt des Verschlussmittel-Tapes 48 wird mittels eines U-förmigen Haltetapes zwischen dessen Schenkeln fixiert. Der in der Ebene darüber hinausstehende Abschnitt 56 trägt eine Kleberbeschichtung mit einem Flächenauftragsgewicht von 38 g/m² ± 4 g/m². Es wird ein an sich üblicher Isopren-Styrol-Copolymer haltiger Haftkleber verwendet. Wie aus der Figur ersichtlich, wird das Tape 48 auf das Testsubstrat 46 aufgebracht und unter einem Anpressdruck von 2,0 kg mit einer automatisierten Anrolleinrichtung bei einer Geschwindigkeit von 300 mm/min überrollt und dadurch angedrückt (hierfür kann eine Anrolleinrichtung vom Typ Viehoever RDG-002-291 verwandt werden).

Über einen verhältnismäßig kurzen Einspannabschnitt wird dann der Verbund aus Stahlplatte 50, Klebeband 52 und Testsubstrat 46 in die eine Klemme 42 des Zugprüfgeräts und das U-förmige Haltetape 54 in die andere Klemme 44 eingespannt. Unter einem Abzugswinkel von im wesentlichen 0°, also in der Ebene des Flachmaterialverbunds, wird dann die bewegliche Klemme 44 mit einer Geschwindigkeit von insbesondere 300 mm/min von der feststehenden wegbewegt. Dabei wird die zwischen den Klemmen auftretende Zugkraft gemessen und in Newton auf zwei Dezimalen gerundet und unter Angabe der Probenbreite, die vorzugsweise 29 mm betragen kann, angegeben. Bei den nachfolgend wiedergegebenen Messwerten handelt es sich jeweils um die Mittelwerte von sechs Einzelmessungen, wobei verschieden dimensionierte Flächen der aktiven Haftverbindung gewählt wurden, deren Abmessung aus den nachfolgend wiedergegebenen Tabellen ersichtlich sind. Die Breite der verwandten Tapes 48, die in der Windel in Querrichtung 28 erstreckt ist, beträgt bei allen Messungen 29 mm. Die als Länge angegebene und variierende Abmessung wäre dann in der Windel in Längsrichtung 30 orientiert. Man erkennt aus der rechten Spalte, dass das Verhältnis der Erstreckung in Längsrichtung 30 (Länge) der htiftnah (oberes Tape) und hüftfern (unteres Tape) angeordneten Verschlussmittel 24, 26 zwischen 1,2 und 2,0 liegt. Entsprechendes gilt dann auch für das Verhältnis der Fläche der aktiven Haftbereiche. Man erkennt, dass auch die gemessenen Scherkräfte, welche die Ablösekräfte, also diejenige Kraft bezeichnen, bei der es quasi schlagartig zu einem Ablösen der Haftverbindung kommt, nahezu exakt dasselbe Verhältnis zeigen.

Die beschriebene Methode stellt daher eine Möglichkeit dar, Ablösekräfte, und zwar als maximale Scherkräfte unter einem Abzugswinkel von 0°, anzugeben oder Ablösekräfte bei verschiedenen Verschlussmittelanordnungen zu vergleichen.

| **Beispiel #** | **Länge oberes Tape [mm]** | **Länge unteres Tape [mm]** | **Verhältnis untere Tapelänge/obere Tapelänge** |
|---|---|---|---|
| 1 | 25 | 35 | 1,4 |
| 2 | 20 | 28 | 1,4 |
| 3 | 25 | 50 | 2,0 |
| 4 | 30 | 42 | 1,4 |
| 5 | 25 | 40 | 1,6 |
| 6 | 25 | 30 | 1,2 |

| **Beispiel #** | **Scherkräfte F**_{**max**}*** oberes Tape [N]** | **Scherkräfte F**_{**max**}*** unteres Tape [N]** | **Verhältnis Scherkräfte F**_{**max**}*** unteres Tape / oberes Tape** |
|---|---|---|---|
| 1 | 48,2 | 66,5 | 1,4 |
| 2 | 39,9 | 55,0 | 1,4 |
| 3 | 48,2 | 96,1 | 2,0 |
| 4 | 57,3 | 79,0 | 1,4 |
| 5 | 48,2 | 75,2 | 1,6 |
| 6 | 48,2 | 57,3 | 1,2 |

| | | | |
|---|---|---|---|
| * Mittelwert der Fₘₐₓ-Werte aus n=6 Messungen | | | |

## Patentansprüche

1. Wegwerfbare Inkontinenzwindel (2) für Erwachsene, mit einem Vorderbereich (6), einem Rückbereich (4) und einem in einer Längsrichtung (30) dazwischen liegenden im Schrittbereich des Benutzers zu liegen kommenden Mittelbereich (8), und mit mechanisch oder klebend wirkenden Verschlussmitteln (24, 26), die an Seitenabschnitten (22, 24), insbesondere an Längsrandabschnitten (18, 20) des Rückbereichs (4) oder des Vorderbereichs (6) angeordnet sind und zum Schließen der Windel (2) mit einem Auftreffabschnitt (32, 34) am Vorderbereich (6) oder Rückbereich (4) haftend zusammenwirken, wobei an einem jeweiligen Seitenabschnitt (22, 24) oder Längsrandabschnitt (18, 20) wenigstens zwei Verschlussmittel (24, 26) vorgesehen sind, die in Längsrichtung (30) voneinander beabstandet sind, **dadurch gekennzeichnet, dass** das jeweilige vom Hüftrand (16) weiter entfernt angeordnete Verschlussmittel (26) und der diesem zugeordnete Auftreffabschnitt (34) so ausgebildet sind, dass sich ihre Haftverbindung ausgehend vom geschlossenen Zustand der Windel (2) erst bei einer höheren Kraft löst als die Haftverbindung bei dem näher am Hüftrand (16) vorgesehenen Verschlussmittel (24) und seinem zugeordneten Auftreffabschnitt (32).

2. Inkontinenzwindel nach Anspruch 1, **dadurch gekennzeichnet, dass** das vom Hüftrand (16) weiter entfernt angeordnete Verschlussmittel (26) einen flächenmäßig größeren aktiven Haftbereich aufweist als das näher am Hüftrand (16) angeordnete Verschlussmittel (24) .

3. Inkontinenzwindel nach Anspruch 1 oder 2, **dadurch gekennzeichnet, dass** die größere Fläche des aktiven Haftbereichs des weiter vom Hüftrand (16) entfernt angeordneten Verschlussmittels (26) wenigstens das 1,2-Fache der Fläche des aktiven Haftbereichs des näher am Hüftrand (16) angeordneten Verschlussmittels (24) beträgt.

4. Inkontinenzwindel nach Anspruch 3, **dadurch gekennzeichnet, dass** die größere Fläche des Haftbereichs wenigstens das 1,2-Fache bis höchstens das 2-Fache, insbesondere bis höchstens das 1,6-Fache und vorzugsweise bis das 1,5-Fache der Fläche des Haftbereichs des näher am Hüftrand (16) angeordneten Verschlussmittels (24) beträgt.

5. Inkontinenzwindel nach einem oder mehreren der vorstehenden Ansprüche, **dadurch gekennzeichnet, dass** die Abmessung des aktiven Haftbereichs des vom Hüftrand (16) weiter entfernt angeordneten Verschlussmittels (26) in Längsrichtung (30) der Windel größer ist als die Abmessung des aktiven Haftbereichs des näher am Hüftrand (16) angeordneten Verschlussmittels (24) in dieser Längsrichtung (30).

6. Inkontinenzwindel nach einem oder mehreren der vorstehenden Ansprüche, **dadurch gekennzeichnet, dass** die Abmessung des aktiven Haftbereichs eines Verschlussmittels (24, 26) in Längsrichtung (30) der Windel im wesentlichen der Abmessung dieses Verschlussmittels (24, 26) in dieser Längsrichtung (30) entspricht.

7. Inkontinenzwindel nach einem oder mehreren der vorstehenden Ansprüche, **dadurch gekennzeichnet, dass** die Abmessung des vom Hüftrand (16) weiter entfernt angeordneten Verschlussmittels (26) in Längsrichtung (30) der Windel größer ist als die Abmessung des näher am Hüftrand (16) angeordneten Verschlussmittels (24) in dieser Richtung (30).

8. Inkontinenzwindel nach einem oder mehreren der vorstehenden Ansprüche, **dadurch gekennzeichnet, dass** die Abmessung des aktiven Haftbereichs des vom Hüftrand (16) weiter entfernt angeordneten Verschlussmittels (26) in Querrichtung (28) der Windel im wesentlichen der Abmessung des aktiven Haftbereichs des näher am Hüftrand (16) angeordneten Verschlussmittels (24) in Querrichtung (28) entspricht.

9. Inkontinenzwindel nach einem oder mehreren der vorstehenden Ansprüche, **dadurch gekennzeichnet, dass** das vom Hüftrand (16) weiter entfernt angeordnete Verschlussmittel (26) eine Abmessung in Längsrichtung (30) der Windel von 24 - 55 mm und das näher am Hüftrand (16) angeordnete eine Abmessung von 20 - 35 mm aufweist.

10. Inkontinenzwindel nach Anspruch 9, **dadurch gekennzeichnet, dass** das vom Hüftrand (16) weiter entfernt angeordnete Verschlussmittel (26) eine Abmessung von 30 - 40 mm und das näher am Hüftrand (16) angeordnete (24) eine Abmessung von 20 - 30 mm aufweist.

11. Inkontinenzwindel nach einem oder mehreren der vorstehenden Ansprüche, **dadurch gekennzeichnet, dass** der Abstand des näher am Hüftrand (16) angeordneten Verschlussmittels (24) vom Hüftrand (16) 10 - 50 mm in Längsrichtung (30) beträgt.

12. Inkontinenzwindel nach einem oder mehreren der vorstehenden Ansprüche, **dadurch gekennzeichnet, dass** der Abstand (h) der zwei Verschlussmittel (24, 26) voneinander 70 - 150 mm in Längsrichtung (30) beträgt.

13. Inkontinenzwindel nach einem oder mehreren der vorstehenden Ansprüche, **dadurch gekennzeichnet, dass** die Ablösekraft, bei der sich die Haftverbindung zwischen dem weiter vom Hüftrand (16) entfernt angeordneten Verschlussmittel (26) und dem zugeordneten Auftreffabschnitt (34) löst - gemessen als bei einem Abzugversuch zwischen Verschlussmittel (26) und Auftreffabschnitt (34) auftretende maximale Scherkraft - wenigstens 45 N beträgt.

14. Inkontinenzwindel nach Anspruch 13, **dadurch gekennzeichnet, dass** die Ablösekraft höchstens 105 N beträgt.

15. Inkontinenzwindel nach Anspruch 14, **dadurch gekennzeichnet, dass** die Ablösekraft zwischen 55 N und 95 N beträgt.

16. Inkontinenzwindel nach einem oder mehreren der vorstehenden Ansprüche, **dadurch gekennzeichnet, dass** die Ablösekraft, bei der sich die Haftverbindung zwischen dem näher am Hüftrand (16) angeordneten Verschlussmittel (24) und dem zugeordneten Auftreffabschnitt (32) löst - gemessen als bei einem Abzugversuch zwischen Verschlussmittel (24) und Auftreffabschnitt (32) auftretende maximale Scherkraft - wenigstens 38 N beträgt.

17. Inkontinenzwindel nach Anspruch 16, **dadurch gekennzeichnet, dass** die Ablösekraft höchstens 60 N beträgt.

18. Inkontinenzwindel nach Anspruch 17, **dadurch gekennzeichnet, dass** die Ablösekraft zwischen 40 N und 55 N beträgt.

## Claims

1. Disposable incontinence pad (2) for adults, comprising a front region (6), a back region (4) and a central region (8) which is located therebetween in a longitudinal direction (30) and which comes to rest in the crotch region of the user, and comprising mechanically or adhesively acting fastening means (24, 26), which are arranged on lateral portions (22, 24), in particular on longitudinal edge portions (18, 20) of the back region (4) or the front region (6) and cooperate adheringly with an abutment portion (32, 34) on the front region (6) or the back region (4) for fastening the pad (2), at least two fastening means (24, 26), which are spaced apart in the longitudinal direction (30), being provided on a respective lateral portion (22, 24) or longitudinal edge portion (18, 20), **characterised in that** the respective fastening means (26), which is arranged further away from the hip edge (16), and the abutment portion (34) associated therewith are configured such that, starting from the fastened state of the pad (2), their adherent connection only becomes detached when it is subjected to a greater degree of force than the adherent connection at the fastening means (24) provided closer to the hip edge (16) and its abutment portion (32) associated therewith.

2. Incontinence pad according to claim 1, **characterised in that** the fastening means (26) arranged further away from the hip edge (16) comprises a larger active adherent region in terms of surface area than the fastening means (24) arranged closer to the hip edge (16).

3. Incontinence pad according to either claim 1 or claim 2, **characterised in that** the larger surface area of the active adherent region of the fastening means (26) arranged further away from the hip edge (16) is at least 1.2 times the surface area of the active adherent region of the fastening means (24) arranged closer to the hip edge (16).

4. Incontinence pad according to claim 3, **characterised in that** the larger surface area of the adherent region is at least 1.2 times to at most 2 times, in particular to at most 1.6 times and preferably to 1.5 times the surface area of the adherent region of the fastening means (24) arranged closer to the hip edge (16).

5. Incontinence pad according to one or more of the preceding claims, **characterised in that** the dimensions of the active adherent region of the fastening means (26) arranged further away from the hip edge (16) are larger in the longitudinal direction (30) of the pad than the dimensions of the active adherent region of the fastening means (24) arranged closer to the hip edge (16) in this longitudinal direction (30).

6. Incontinence pad according to one or more of the preceding claims, **characterised in that** the dimensions of the active adherent region of a fastening means (24, 26) in the longitudinal direction (30) of the pad substantially correspond to the dimensions of this fastening means (24, 26) in this longitudinal direction (30).

7. Incontinence pad according to one or more of the preceding claims, **characterised in that** the dimensions of the fastening means (26) arranged further away from the hip edge (16) are larger in the longitudinal direction (30) of the pad than the dimensions of the fastening means (24) arranged closer to the hip edge (16) in this direction (30).

8. Incontinence pad according to one or more of the preceding claims, **characterised in that** the dimensions of the active adherent region of the fastening means (26) arranged further away from the hip edge (16) substantially correspond in the transverse direction (28) of the pad to the dimensions of the active adherent region of the fastening means (24) arranged closer to the hip edge (16) in the transverse direction (28).

9. Incontinence pad according to one or more of the preceding claims, **characterised in that** the fastening means (26) arranged further away from the hip edge (16) has dimensions in the longitudinal direction (30) of the pad of 24 to 55 mm and that arranged closer to the hip edge (16) has dimensions of 20 to 35 mm.

10. Incontinence pad according to claim 9, **characterised in that** the fastening means (26) arranged further away from the hip edge (16) has dimensions of 30 to 40 mm and that (24) arranged closer to the hip edge (16) has dimensions of 20 to 30 mm.

11. Incontinence pad according to one or more of the preceding claims, **characterised in that** the distance of the fastening means (24), which is arranged closer to the hip edge (16), from the hip edge (16) is 10 to 50 mm in the longitudinal direction (30).

12. Incontinence pad according to one or more of the preceding claims, **characterised in that** the distance (h) of the two fastening means (24, 26) from each other is 70 to 150 mm in the longitudinal direction (30).

13. Incontinence pad according to one or more of the preceding claims, **characterised in that** the detachment force at which the adherent connection between the fastening means (26) arranged further away from the hip edge (16) and the associated abutment portion (34) becomes detached (measured as a maximum shear force occurring during a tear-off test between the fastening means (26) and the abutment portion (34)) is at least 45 N.

14. Incontinence pad according to claim 13, **characterised in that** the detachment force is at most 105 N.

15. Incontinence pad according to claim 14, **characterised in that** the detachment force is between 55 N and 95 N.

16. Incontinence pad according to one or more of the preceding claims, **characterised in that** the detachment force at which the adherent connection between the fastening means (24) arranged closer to the hip edge (16) and the associated abutment portion (32) becomes detached (measured as a maximum shear force occurring during a tear-off test between the fastening means (24) and the abutment portion (32)) is at least 38 N.

17. Incontinence pad according to claim 16, **characterised in that** the detachment force is at least 60 N.

18. Incontinence pad according to claim 17, **characterised in that** the detachment force is between 40 N and 55 N.

## Revendications

1. Couche pour incontinence jetable (2) pour adultes, comprenant une zone avant (6), une zone arrière (4) et une zone centrale (8) située entre les deux dans une direction longitudinale (30), venant se situer dans la zone d'entrejambe de l'utilisateur, et des moyens de fermeture (24, 26) agissant mécaniquement ou en collant, lesquels sont agencés sur des parties latérales (22, 23), en particulier sur des parties de bord longitudinales (18, 20) de la zone arrière (4) ou de la zone avant (6) et coopèrent par adhérence pour fermer la couche (2) avec un segment de contact (32, 34) sur la zone avant (6) ou la zone arrière (4), au moins deux moyens de fermeture (24, 26) étant prévus sur chaque partie latérale (22, 23) ou partie de bord longitudinale (18, 20), lesquels sont distants l'un de l'autre dans la direction longitudinale (30), **caractérisée en ce que** chaque moyen de fermeture (26) agencé le plus loin du bord de hanche (16) et le segment de contact (34) associé à celui-ci sont formés de telle manière que leur liaison par adhérence ne se détache depuis l'état fermé de la couche (2) qu'en cas de force plus grande que la liaison par adhérence entre le moyen de fermeture (24) prévu le plus près du bord de hanche (16) et son segment de contact associé (32).

2. Couche pour incontinence selon la revendication 1, **caractérisée en ce que** le moyen de fermeture (26) agencé le plus loin du bord de hanche (16) présente une zone d'adhérence active plus grande, du point de vue de la surface, que le moyen de fermeture (24) agencé le plus près du bord de hanche (16).

3. Couche pour incontinence selon la revendication 1 ou 2, **caractérisée en ce que** la plus grande surface de la zone d'adhérence active du moyen de fermeture (26) agencé le plus loin du bord de hanche (16) est égale à au moins 1,2 fois la surface de la zone d'adhérence active du moyen de fermeture (24) agencé le plus près du bord de hanche (16).

4. Couche pour incontinence selon la revendication 3, **caractérisée en ce que** la plus grande surface de la zone d'adhérence est comprise entre 1,2 fois au moins et 2 fois au maximum, en particulier 1,6 fois au maximum et de préférence jusqu'à 1,5 fois la surface de la zone d'adhérence du moyen de fermeture (24) agencé le plus près du bord de hanche (16).

5. Couche pour incontinence selon l'une ou plusieurs des revendications précédentes, **caractérisée en ce que** la dimension de la zone d'adhérence active du moyen de fermeture (26) agencé le plus loin du bord de hanche (16) est plus grande dans la direction longitudinale (30) de la couche que la dimension de la zone d'adhérence active du moyen de fermeture (24) agencé le plus près du bord de hanche (16) dans cette direction longitudinale (30).

6. Couche pour incontinence selon l'une ou plusieurs des revendications précédentes, **caractérisée en ce que** la dimension de la zone d'adhérence active d'un moyen de fermeture (24, 26) dans la direction longitudinale (30) de la couche correspond essentiellement à la dimension de ce moyen de fermeture (24, 26) dans cette direction longitudinale (30).

7. Couche pour incontinence selon l'une ou plusieurs des revendications précédentes, **caractérisée en ce que** la dimension du moyen de fermeture (26) agencé le plus loin du bord de hanche (16) est plus grande dans la direction longitudinale (30) de la couche que la dimension du moyen de fermeture (24) agencé le plus près du bord de hanche (16) dans cette direction (30).

8. Couche pour incontinence selon l'une ou plusieurs des revendications précédentes, **caractérisée en ce que** la dimension de la zone d'adhérence active du moyen de fermeture (26) agencé le plus loin du bord de hanche (16) dans la direction transversale (28) de la couche correspond essentiellement à la dimension de la zone d'adhérence active du moyen de fermeture (24) agencé le plus près du bord de hanche (16) dans la direction transversale (28).

9. Couche pour incontinence selon l'une ou plusieurs des revendications précédentes, **caractérisée en ce que** le moyen de fermeture (26) agencé le plus loin du bord de hanche (16) présente dans la direction longitudinale (30) de la couche une dimension comprise entre 24 et 55 mm et celui agencé le plus près du bord de hanche (16) une dimension comprise entre 20 et 35 mm.

10. Couche pour incontinence selon la revendication 9, **caractérisée en ce que** le moyen de fermeture (26) agencé le plus loin du bord de hanche (16) présente une dimension comprise entre 30 et 40 mm et celui (24) agencé le plus près du bord de hanche (16) une dimension comprise entre 20 et 30 mm.

11. Couche pour incontinence selon l'une ou plusieurs des revendications précédentes, **caractérisée en ce que** l'écart entre le moyen de fermeture (24) agencé le plus près du bord de hanche (16) et le bord de hanche (16) dans la direction longitudinale (30) est compris entre 10 et 50 mm.

12. Couche pour incontinence selon l'une ou plusieurs des revendications précédentes, **caractérisée en ce que** l'écart (h) entre les deux moyens de fermeture (24, 26) dans la direction longitudinale (30) est compris entre 70 et 150 mm.

13. Couche pour incontinence selon l'une ou plusieurs des revendications précédentes, **caractérisée en ce que** la force de séparation à laquelle la liaison par adhérence entre le moyen de fermeture (26) agencé le plus loin du bord de hanche (16) et le segment de contact associé (34) se sépare, mesurée en tant que force de cisaillement maximale produite lors d'un essai de traction entre moyen de fermeture (26) et segment de contact (34), est au moins égale à 45 N.

14. Couche pour incontinence selon la revendication 13, **caractérisée en ce que** la force de séparation est égale à 105 N au maximum.

15. Couche pour incontinence selon la revendication 14, **caractérisée en ce que** la force de séparation est comprise entre 55 N et 95 N.

16. Couche pour incontinence selon l'une ou plusieurs des revendications précédentes, **caractérisée en ce que** la force de séparation à laquelle la liaison par adhérence entre le moyen de fermeture (24) agencé le plus près du bord de hanche (16) et le segment de contact associé (32) se sépare, mesurée en tant que force de cisaillement maximale produite lors d'un essai de traction entre moyen de fermeture (24) et segment de contact (32) est d'au moins 38 N.

17. Couche pour incontinence selon la revendication 16, **caractérisée en ce que** la force de séparation est égale à 60 N maximum.

18. Couche pour incontinence selon la revendication 17, **caractérisée en ce que** la force de séparation est comprise entre 40 N et 55 N.
